# EUROPEAN PATENT APPLICATION

(11) **EP 0 647 709 A1**
(43) Date of publication of application: **12.04.1995**
(21) Application number: 94202885.3
(22) Date of filing: 05.10.1994
(51) Int. Cl.: C12N 1/04

(54) **Process for preparing dry compositions of heat-sensitive materials**

(30) Priority: 12.10.1993 EP 93202881
(71) Applicant: UNILEVER N.V., NL-3000 DK Rotterdam (NL); UNILEVER PLC, London EC4P 4BQ (GB)
(72) Inventor: Lievense, Lourus Cornelis, Unilever Research, NL-3133 AT Vlaardingen (NL); Hoogland, Martin, NL-1251 PH Laren (NL)
(74) Representative: Hartong, Richard Leroy

(57) **Abstract**

There is provided a process for preparing a substantially dry and stable composition containing a high concentration of heat-sensitive material, which process comprises:
(a) contacting an aqueous concentrate of the heat-sensitive material with a food-grade water-absorbing material during sufficient time to allow transport of a large proportion of the water from the concentrate to the water-absorbing material,
(b) separating the concentrate having a decreased water content from the water-absorbing material, and
(c) collecting the separated concentrate.
The heat-sensitive material in particular consists of microbial cells and the composition contains at least 10⁷ viable cells per gram of dry and stable composition.

## Description

The present invention relates to a process for preparing a substantially dry and stable composition of heat sensitive materials, in particular a composition containing at least 10⁷ viable microbial cells per gram.

### Background of the invention

Modern industrial processes require starter cultures (yeasts and bacteria) with well-defined properties in order to generate end products with constant quality. Frozen as well as (freeze-)dried formulations are used for this purpose. The main advantage of dried over frozen micro-organisms is the lower transport and storage costs of the cultures. For industrial use, large quantities of active cells are required. Freeze-drying is generally not considered to be an attractive method for the preparation of these large quantities, due to its high cost and inherent complexity.

Potentially, convective drying methods, such as spray-drying and fluidized bed drying, are more economical. However, a major disadvantage of convective drying is that a considerable part of the cells is inactivated during drying due to the required high air temperatures. It is generally assumed this thermal inactivation is the main reason for inactivation during convective drying, although it was shown that the inactivation of *Lb*. *plantarum* during fluidized bed drying was caused by thermal inactivation as well as by the dehydration itself (Lievense et al. 1991). Thus, inactivation during convective drying can be caused by two mechanisms, thermal inactivation and dehydration-inactivation.

The main convective drying methods, i.e. spray-drying, fluidized bed drying and spray granulation, have several advantages and disadvantages among each other. A major advantage of spray-drying is that no additional processing steps are needed, and the concentrated biomass can be dried directly. A disadvantage of spray-drying is that in principle the drying time is fixed by the dimensions of the drier. The specific heat of evaporation must be supplied in a relatively short time and thus high inlet temperatures are required. This can result in a significant thermal inactivation of the cells. Alternatively, fluidized bed and spray granulation drying offer the freedom of choosing the drying time and thus the air temperature. Therefore, thermal inactivation can be minimized. However, these latter processes require a support material and/or additional processing steps.

A method of drying micro-organisms by means of Contact Sorption Drying (CSD) is known from EP-A-298605 (Unilever). The concept of CSD is that moisture from a suspension with a relative high solids content is absorbed by a dry carrier resulting in a low moisture cell/carrier product. In EP-A-298605 a process is described for the preparation of a supported microflora composition of improved ambient stability suitable for use as an inoculum, the constituents of which comprise an inert, subdivided support and an aqueous suspension of viable microflora, whereby the water content and the proportions of each are such that the composition survives ambient storage, in which process an aqueous concentrate of microflora is dispersed on an inert particulate support material, characterised in that the support material is dried beforehand by heating and that the proportion and concentration of the concentrate are selected to provide a composition having a water activity of 0.3 or below.

CSD differs significantly from convective drying because convective drying is based on the removal of water from the product by evaporation. To supply the energy for evaporation, heated air has to be applied. Depending on the micro-organism and air temperature, this can lead to a significant inactivation of the microbial cell. With CSD, thermal inactivation of the cells can be avoided because the water is removed by absorption by the dried carrier. Although CSD can be of major importance in the formulation of bakery products, which are the main subject of EP-A-298605, other uses are limited. This is due to the relatively low concentration of microbial cells and inherent high concentration of carrier material in the final product.

US-A-2,853,797 describes a process for dehydrating a bacterial dispersion by freezing droplets of the dispersion and vacuum drying the frozen particles using silica gel as an absorbent. Disadvantages of this process include the use of silica gel which is not really a food-grade absorbent, and the expensive freezing means.

It is thus an object of the present invention to provide a solution to the problem of the low product concentration of CSD-treated, heat-sensitive materials.

### Summary of the invention

The invention provides a process for preparing a substantially dry and stable composition containing a high concentration of a heat-sensitive material, comprising (a) contacting an aqueous concentrate of the heat-sensitive material with a food-grade water-absorbing material during sufficient time to allow transport of a large proportion of the water from the concentrate to the water-absorbing material, (b) separating the concentrate having a decreased water content from the water-absorbing material, and (c) collecting the separated concentrate.

Step (a) of the process according to the invention corresponds to Contact Sorption Drying, and can be performed e.g. as described in EP-A-298605 with addition methods suitable to provide a significant difference in particle size, density, mass and/or surface between concentrate and absorbent.

Step (b) of the process according to the invention can be carried out by any suitable physical separation technique, in particular separation on the basis of particle size, particle density, particle mass or particle surface, e.g. by sieving, air classification or cyclone separation.

The heat-sensitive material especially consists of microbial cells, which may be moulds, yeasts and bacteria, e.g. lactic acid bacteria, or bacteria having probiotic properties.

The invention also relates to dry and stable compositions that can be prepared by such a process, the compositions containing at least 10⁷ viable microbial cells per gram.

The heat-sensitive material can also be other than microbial cells, such as food ingredients and pharmaceutical and cosmetic ingredients. The dried, stable concentrate preferably contains at least 0.9 gram of such ingredients per gram of such a composition.

### Brief description of the figures

Fig. 1: Yeast activity of fresh and dried yeast (air-jet sieve fractions)
Fig. 2: Average weight and yeast count distribution (vibration sieve fractions)
Fig. 3: Storage stability of processed yeast - Viable count
Fig. 4: Storage stability of processed yeast - Activity
Fig. 5: Storage stability of processed *Lb*. *plantarum* - Viable count
Fig. 6: Storage stability of processed *Lb*. *plantarum* -Activity
Fig. 7: Layout of the sorption-separation process

### Detailed description of the invention

According to the present invention, the mild technique of drying by absorption of moisture, and thus preventing thermal inactivation of heat-labile components, is combined with the advantage that before drying no further processing steps are needed, i.e. the heat-sensitive material can directly be added to the absorbent. The absorbent is removed in the separating step after a certain drying time and the dried product can be obtained in a high concentration. For example, the agglomerated dried cell droplets can be separated from the mixture by sieving. In this way the viable count of the final compositions so obtained is significantly improved, which largely extends the range of possible applications of these compositions.

In the present specification, a substantially dry composition is understood to be a composition containing less than 50 wt.% of free water, preferably less than 20 wt.% of free water (or 0.25 kg H₂O per kg of dry solids), most preferably less than 10 wt.% of free water (or 0.11 kg H₂O per kg of dry solids). The transport of a large proportion of the water from the aqueous concentrate to the absorbing material means the transport of at least 75%, preferably at least 90% of the water originally present in the concentrate to the absorbent.

Various water-absorbing materials can be used in step (a). Suitable water-absorbing materials are food-grade and poorly soluble, preferably insoluble, in water. A material is considered to be food-grade if it can be combined with foodstuffs for human consumption in appreciable quantities without health risks. The water-absorbing materials are preferably selected from bulk agricultural products or derivatives thereof. Examples of bulk agricultural products are: cereals, tuberous plants, peas, beans and milk, and derivatives thereof. Suitable absorbents are mentioned in EP-A-298605. These include polysaccharide-based and/or protein-based absorbents. Suitable examples of polysaccharide-based absorbents include wheat, rice, barley, rye, oat, buckwheat, maize, tapioca, soybean, (sweet) potato, sorghum, millet, cassave (manioc) and sunflower flour and derivatives thereof. Examples of derivatives include starch and starch hydrolysis products, gluten, cellulose, fibres, carboxymethylcellulose, oligofructoses, polydextroses, milk and whey powder. Other suitable absorbents include natural polymers as food hydrocolloids and stabilisers like carrageenan, galactomannans, glucomannans, guar gum, arabic gum, locust bean gum, xanthan gum, pectin and alginate. Semi-synthetic and synthetic absorbents can also be used, provided that they do not pose a problem in the final use, e.g. with respect to food compatibility or the heat-sensitivity of the material to be dried.

The water-absorbing material should preferably have a wet density or a wet particle size that is appreciably different from the density or particle size of the dried heat-sensitive material. For example, when sieving is used as the separation technique of step (b) of the process of the invention, the particle size of the wet absorbing material may advantageously be smaller than the particles of the heat-sensitive material, e.g. microbial cell concentrate, so that the absorbing material can pass the sieve and the cells are retained.

When, for example, cyclone separation or air classification is used as the separation technique of step (b), the particle surface/density of the wet absorbing material may advantageously be significantly different from the particle surface/density of the dried heat-sensitive material. In this way, an efficient separation can be achieved. The process used for the addition of the heat-sensitive material to the absorbing material is important for the efficiency of the later separation step, as will be illustrated in Example 4.

The heat-sensitive materials may be protected during the absorption and separation steps of the present process by means of protective materials, such as mono- and di-saccharides or polysaccharides. Such protective materials may also serve to adapt the particle size or the viscosity of the materials in view of the separation step. For example, bacterial cell preparations may be protected by means of sucrose, as is known in the art.

An aqueous dispersion or concentrate is understood to be a dispersion in water, optionally with a protectant, which is in the liquid state; this may include supercooled slurries. Contacting of the aqueous concentrate with the absorbent is preferably carried out by addition of the dispersion, more in particular by dropwise addition.

In case of microbial cell compositions, the cell compositions obtained using the process according to the invention contain at least 10⁶, more preferably at least 10⁷, most preferably at least 10⁸ viable cells per g of the composition.

The present invention allows a considerable concentration of the biomass as heat-labile material, up to 55 times compared to the CSD process (EP-A-298605). This considerably extends the possible uses of this technology. With the combination of the selection of dehydration resistant strains and the mild drying conditions of the present process, high survival rates of bacterial cells can be obtained (up to 100%). Due to the mild temperatures, thermal inactivation of the micro-organisms, additional to dehydration inactivation, will not occur in the present process. The main advantage of the sorption/separation process of the present invention is that the concentrated biomass can be dried directly without the need of additional processing steps. This is in contrast to e.g. convective fluidized bed drying, which can also be carried out at low air temperatures, but which requires mixing and extrusion in order to obtain a granulate (Lievense and Van 't Riet 1993). Using the process according to the invention, products could be obtained that were stable for at least 6 months at ambient conditions.

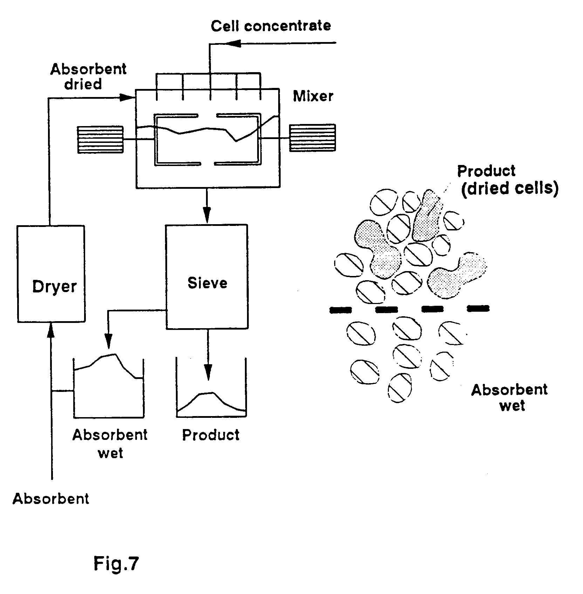

It is possible in the commercial use of the present invention to recycle the absorbent, i.e. drying the used wet absorbent in an additional drying step. If this drying step is carried out in a fluidized bed drier, this drier can also be used for further drying the biomass pellets. A diagram for this process involving regeneration of the sorbent is given in Fig. 7. Another possibility is to sell the spent absorbent for other purposes e.g. animal feed.

### Example 1: Sorption/separation of yeast concentrate

### Biomass preparation

*Saccharomyces cerevisiae*, was freshly obtained as commercially available active pressed yeast (Koningsgist, Gist-brocades, NL). The pressed yeast was mixed in equal amounts (w/w) with sucrose as a fine powder. Addition of sucrose (0.02 kg H₂O/kg dry solids) to *S*. *cerevisiae* (2.66 kg H₂O/kg dry solids) led to osmotic dehydration of the yeast cells and to liquification of the solid pressed yeast, which was necessary to make the pressed yeast pumpable.

### Contact Sorption Drying

An amount of 25 g yeast-sucrose suspension was added dropwise with a Masterflex pump and a hollow needle to 500 g flour as absorbent (Columbus wheat flour, Meneba, NL), which was pre-dried in a fluidized bed drier at 100°C for 1 h, to a final moisture level of less than 0.02 kg H₂O/kg dry solids. The flour as absorbent was placed in a mixer (Primax AK5, Küpper-Primax, Troisdorf, Germany) and stirred at 50 rpm. The suspension was pumped with a velocity of 3.3 g/min, and dropped through a hollow needle (⌀ 0.5 mm) to provide a significant difference in particle size of the final biomass particle and the absorbent. After the whole yeast/sucrose suspension was added, the absorbent/suspension mixture was additionally mixed for 1 h.

### Sieving

The dried yeast-sucrose droplets, so called pellets, were separated from the mixture by sieving. Two types of laboratory size sieving equipment were applied: A vibration sieve (Engelsmann AG, Ludwigshafen, Germany) or an air-jet sieve (Alpine 200LS, Augsburg, Germany). For a standard separation of the pellets and the absorbent and unless mentioned otherwise, 100 g of the total mixture of 525 g, was sieved in the vibration sieve (sieve-mesh of 0.355 mm) for 15 min, or in the air-jet sieve (sieve-mesh of 0.140 mm) for 3 min. The pellets were collected in the sieve, while the absorbent was collected in the container of the vibration sieve or in the dust bag in the vacuum cleaner of the air-jet sieve.

### Activity Measurement

For *S*. *cerevisiae*, the metabolic activity was estimated in an application test in which the proofing of a dough was measured. 13.2 g of Columbus wheat flour (Meneba, NL) was mixed with 0.55 g fresh pressed yeast (containing approximately 30% dry weight of yeast), 0.25 g of NaCl and 10.0 g of water. The dough was kneaded manually for 5 min, whereafter the dough was transferred in a 100 ml pre-warmed measuring cylinder at 30°C, pushed flat and incubated in a stove at 30°C. The volume of the dough was recorded every 5-10 min (no correction was made for the slightly round dough surface). The activity of the yeast is expressed as the time required for the dough to double its volume. Before measuring the activity of the dried yeast, the pellets were rehydrated for 5 min at 5°C in water. The activity measurement before drying was carried out with the same components in the same amounts in the system as present at measurement of the activity of the dried pellets (e.g. equivalent amounts of biomass, absorbent and sucrose).

### Viable Counts

Viable cell numbers of pressed yeast, wet and dried yeast-sucrose suspensions were counted by incubating in Malt-agar (Difco #B24) for 48 h at 30°C. In all experiments the survival rate after drying and after storage is calculated as the ratio between the total number of viable cells (Colony Forming Units (CFU)) before and after drying.

**Table 1**

| Summary of results for processed yeast | | | | | | | | |
|---|---|---|---|---|---|---|---|---|
| Exp no | added biomass CFU/g (x10⁹) | sieve (1) | g pellet/100 g mix (4) | CFU/g product (x10⁸) | product water g/g tot | survival % CFU | activity DD in min (2) | |
| | | | | | | | fresh | dry |
| 1 | 6.2 | vib | 40.6 | 20 | .064 | 110 | nd | |
| 2 | 6.3 | vib | 34.8 | 2.9 | .046 | 65 | nd | |
| 3 | 11 | vib | 14.8 | 7.8 | .095 | 46 | nd | |
| 4 | 6.7 | vib | 23.0 | 8.2 | .082 | 120 | nd | |
| 5 | 6.1 | vib | 87.6 | 1.5 | .029 | 85 | nd | |
| 6 | 6.9 | air | 17.7 | 17 | .076 | 58 | 37 | - |
| 7 | 7.0 | air | 16.8 | 5.8 | .071 | 55 | 40 | 50 |
| 8 (3) | 9.0 | air | 17.6 | 5.0 | .055 | 42 | 37 | 53 |

| | | | | | | | | |
|---|---|---|---|---|---|---|---|---|
| (1) DD = dough doubling time in min | | | | | | | | |
| (2) vib = vibration sieve (mesh 0.355 mm); air = air-jet sieve (mesh 0.125 mm) | | | | | | | | |
| (3) used in storage stability test | | | | | | | | |
| (4) mix = total mixture of absorbent and yeast suspension with sucrose nd = not determined | | | | | | | | |

### Survival of the yeast cells

As can be seen from Table 1, the survival of pressed yeast in this process varies considerably. This is probably due to the different batches of fresh yeast used and the variation caused by the plate count method. The physiological state of the yeast cells (time after harvesting) and thus drying resistance will differ from batch to batch, due to fermentation and storage conditions. Nevertheless, the survival based on CFU is reasonable, varying from 40 to 100%. If a drying resistant yeast strain is used which is cultured under appropriate conditions and with a defined physiological state (i.e. defined culture age), the average survival will probably increase and be more consistent.

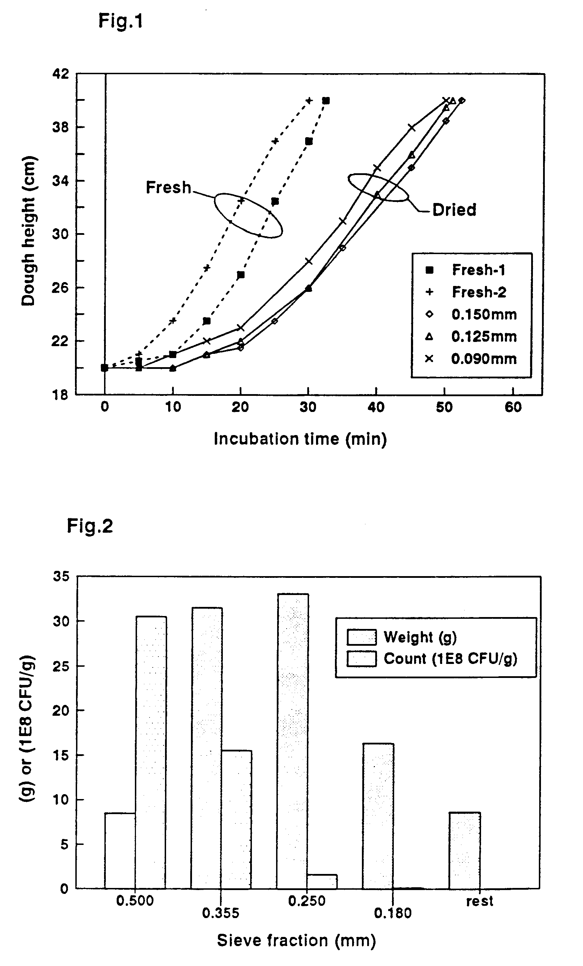

In Fig. 1 the yeast activity of pellets from different sieve fractions and the activity of fresh yeast is plotted in time. After a rehydration time of the pellets of 5 min, the lag-time of the dried yeast is significant. This lag-time can only be reduced by selection of the proper drying resistant yeast strain which is cultured under appropriate conditions.

### Example 2: Sorption/separation of bacterial concentrate

### Biomass preparation

*Lactobacillus plantarum* Vl342 was grown as a stirred 2 L culture for ca. 15 h at 36 °C and pH 6.3 using MRS (Difco), fortified with 20 g/l glucose and 2 g/l yeast extract under micro-aerophilic conditions. The pH was adjusted with NaOH. Cultivation was stopped at about 2 h after the culture reached the stationary growth phase. The final culture was centrifuged for 20 min at 9000 rpm (9250-10450 g) at 5°C. After discarding the supernatants, cell pellets were collected and immediately processed further. Different methods for preparing the biomass for sorption/separation, and three different absorbent materials were applied in different experiments (see below). If sucrose was used as a protectant, the cell pellets were mixed in equal amounts (w/w) with sucrose as a fine powder. Addition of sucrose (0.02 kg H₂O/kg dry solids) to a pellet of *Lb*. *plantarum* (3.39 kg H₂O/kg dry solids) leads to osmotic dehydration of the cells and to liquification of the solid pellet. The cell pellets of *Lb*. *plantarum* dried without sucrose were diluted with physiological salt (PS) solution (0.9% NaCl in demi-water) (ratio biomass:PS-solution ranging from 3:1 to 4:1), which was necessary to make the biomass pumpable.

### Contact Sorption Drying and sieving

Three different absorbents were used, namely flour (Columbus wheat flour, Meneba, NL), NFDM (Non Fat Dry Milk powder Grade A, Maryland & Virginia Milk Products Ass., Laurel, Maryland, USA) and starch (Tapioca starch, National Starch & Chemical, Germany). All absorbents were pre-dried in a fluidized bed drier at 100°C for 1 h, to a final moisture level of less than 0.02 kg H₂O/kg dry solids. An amount of about 20 g *Lb*. *plantarum* suspension (with or without sucrose) was added dropwise (3.1 g/min) to 500 g absorbent and processed further as described in Example 1. The dried pellets were separated from the mixture by air-jet sieving as described in Example 1.

### Activity Measurement

The activity of a cell suspension of *Lb*. *plantarum* was measured in a model test with potassium phosphate buffer (KPi-buffer) and is based on the glucose-fermenting activity of a non-growing cell suspension (Lievense et al. 1990). Before measuring the activity of the dried biomass, the pellets were rehydrated for 5 min at 5°C in KPi-buffer. In all the activity measurements where a residual activity (actdry/actwet) is defined, the activity measurement before drying (wet) was carried out with the same components in the same amounts in the system as present at measurement of the activity of the dried pellets (e.g. equivalent amounts of biomass, absorbent and sucrose).

### Viable Counts

Viable cell numbers of concentrated cells, processed and dried cell suspensions of *Lb*. *plantarum* were determined after incubation for 48 h at 37°C under aerobic conditions using MRS-agar (Difco). In all experiments the survival rate after drying and after storage is calculated as the ratio between the total number of viable cells (CFU) before and after drying.

### Survival of the bacterial cells

As can be seen from Table 2, the survival of *Lb*. *plantarum* is generally high. Addition of sucrose to the pellets of *Lb*. *plantarum* did not have a significant positive effect on the drying resistance. However, in storage stability experiments with CSD-mixtures (EP-A-298605), it was shown that sucrose significantly increased stability during storage. For different uses of *Lb*. *plantarum*, different absorbents can be applied. As can be concluded from these experiments, the nature of the absorbent material used did not influence the residual activity after drying.

**Table 2**

| Summary of results of processed *Lb*. *plantarum* | | | | | | | | | |
|---|---|---|---|---|---|---|---|---|---|
| Exp no | added biomass CFU/g (x10¹¹) | sucrose | absorbent (2) | sieve (1) | g pellet/100 g mix (4) | CFU/g product (x10¹¹) | product water g/g | survival % | |
| | | | | | | | | CFU | act |
| 1 | 1.5 | + | F | air | 13.0 | 0.17 | .053 | 72 | 83 |
| 2 | 4.5 | + | N | air | 8.3 | 1.3 | .104 | 98 | 120 |
| 3 (3) | 3.0 | + | F | air | 10.3 | 0.8 | .080 | 120 | 100 |
| 4 | 3.2 | - | S | air | 1.8 | 6.3 | .160 | 98 | 111 |
| 5 | 3.0 | - | S | air | 1.7 | 4.4 | .130 | 76 | 103 |

| | | | | | | | | | |
|---|---|---|---|---|---|---|---|---|---|
| (1) air = air-jet sieve (mesh 0.140 mm) | | | | | | | | | |
| (2) F = flour; N = NFDM; S = starch | | | | | | | | | |
| (3) used in storage stability tests | | | | | | | | | |
| (4) mix = total mixture of absorbent and Lb. plantarum suspension with or without sucrose | | | | | | | | | |

### Example 3: Difference in vibration and air-jet sieving

In Fig. 2 the size distribution of several sieve fractions of a mixture prepared according to Example 1 and the yeast concentration in each fraction are given. As can be seen from Fig. 2, nearly all the yeast cells are present in the sieve fractions ≧ 0.355 mm. This means that a significant concentration of the dried biomass can be achieved by sieving without a considerable biomass loss. With vibration sieving, the viable yeast count in the dried product could on average be increased from 1.9 (±1.3) * 10⁸ CFU/g in the mix to 8.1 (±6.6) * 10⁸ CFU/g in the pellets, which means an average concentration factor of 3.6. The average mass concentration factor for the same experiments is 3.5. Due to blocking of the sieve, which was dependent on relative air humidity and absorbent, vibration sieving gave unreproducible results, regarding the amount of pellets that remained in the sieve (concentration factor) and thus the count in the final product. The differences in physiological state of the yeast cells, and thus the survival of the yeast, causes a variation in the product count as well (see Example 1).

To obtain a more constant concentration factor, air-jet sieving was applied. The air stream of the air-jet sieve prevents blocking of the sieve and partly removes the absorbent adhered to the pellets, which leads to a higher concentration factor, smaller pellets, and a shorter sieving time. The product yeast CFU of the air-jet sieved fractions was increased by an average factor of 5.1, the average mass concentration factor was about the same. For most of the separations performed with the air-jet sieve, a mesh of 0.125-0.140 mm was found to be appropriate versus 0.355 mm for the vibration sieve.

The maximum mass concentration factor achieved with air-jet sieving was around 55, in experiments performed with *Lb*. *plantarum*. Air-jet sieving of 100 g of cell/absorbent mix resulted in 1.7-1.8 g of product with an average count of 5.4*10¹¹ CFU/g (see Table 2). This is 75% of the maximally achievable viable count based on the amount of cells added to the total mixture.

### Example 4: Importance of heat-sensitive material to absorbent addition process

The importance of the process of addition of the biomass slurry to the absorbent on the final separation step was also investigated. A two-phase spray-nozzle (of the laboratory spray-dryer Büchi 190, Flawil, CH) was used to spray 21.6 g *Lb*. *plantarum* biomass slurry on 500 g pre-dried tapioca starch (see also Example 2). *Lb*. *plantarum* Vl342 was grown and concentrated as described in Example 2.

A dilution of biomass (without sucrose) with the PS-solution of 1.1:1 was required to make the spraying possible. In Experiment 2, in which a hollow needle for the biomass addition was used, a biomass:PS-solution ratio of 3:1 to 4:1 could be applied. This extra required dilution is a disadvantage of the spraying method compared to the addition method used in Example 2. Because of the higher dilution, more water is introduced and more absorbent will be required to dry an equivalent amount of biomass.

An even more important disadvantage of biomass addition by spraying is the low efficiency of the subsequent separation step, which is performed in the same way as the separation described in Example 2. The amount of pellets separated from the total biomass-absorbent mixture with their viable cell counts is given in Table 3, together with the viable cell count of the separated absorbent. Only 45% of the total present viable counts could be separated form the biomass-absorbent mixture when spraying through a two-phase nozzle (Exp. 6) was used as the addition method, versus a 99% separation efficiency when dropwise addition, e.g. through a hollow needle, (Exp. 4) was applied. The survival of the *Lb*. *plantarum* cells was not influenced by the difference in addition method.

**Table 3**

| Influence of biomass addition process | | | | | | | | | |
|---|---|---|---|---|---|---|---|---|---|
| Exp no | suspension ratio pellet t/PS | added biomass CFU/g (x10¹¹) | g susp. added per 100 g absorbent | g pellet/100 g mix | CFU/g product (x10¹¹) | CFU/g rest absorbent (x10⁷) | survival % CFU | % CFU in | |
| | | | | | | | | pellet | rest |
| 4 | 3.35 | 3.2 | 3.60 | 1.8 | 6.3 | 6.6 | 98 | 99 | 1 |
| 6 | 1.07 | 1.6 | 4.32 | 4.2 | 0.7 | 371 | 97 | 45 | 55 |

### Example 5: Storage stability of processed products

With dried samples of *Lb*. *plantarum* and *S*. *cerevisiae* storage stability tests were carried out. Dried pellets (4.0 g for *Lb*. *plantarum* and 5.1 g for *S*. *cerevisiae*) were hermetically packed under atmospheric conditions (air) using 100 ml glass jars (twist-off lid) and stored in incubators at 5°C and 25°C for up to 7 months. At different times, metabolic activities and viable counts were measured.

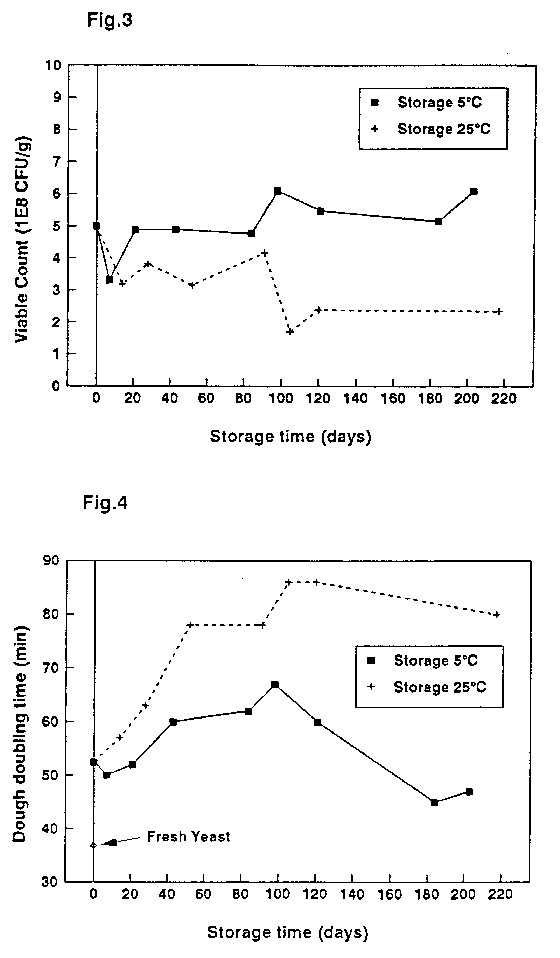

In Fig. 3 and Fig. 4, the stability of yeast dried on Columbus flour and separated with the air-jet sieve is shown. In Fig. 3 the viable yeast count in the pellets stored at 5 and 25°C for a period of 218 days is given. The count remains stable at 5°C but decreases slightly at 25°C. This can also be observed in Fig.4 where the yeast activity (dough doubling time) is given as a function of storage time. The activity of an equal amount of fresh yeast is also given in Fig. 4. The significant lag-phase of dried yeast causes an increase in dough doubling time (decrease in activity) already directly after drying (see also Fig. 1). Why the activity increases after a certain storage time at 5°C is not clear. This can be due to inhomogeneity in the stored pellets (i.e. the batches of stored pellets contain different amounts of active yeast cells). However, this is not clear from the viable count measurements.

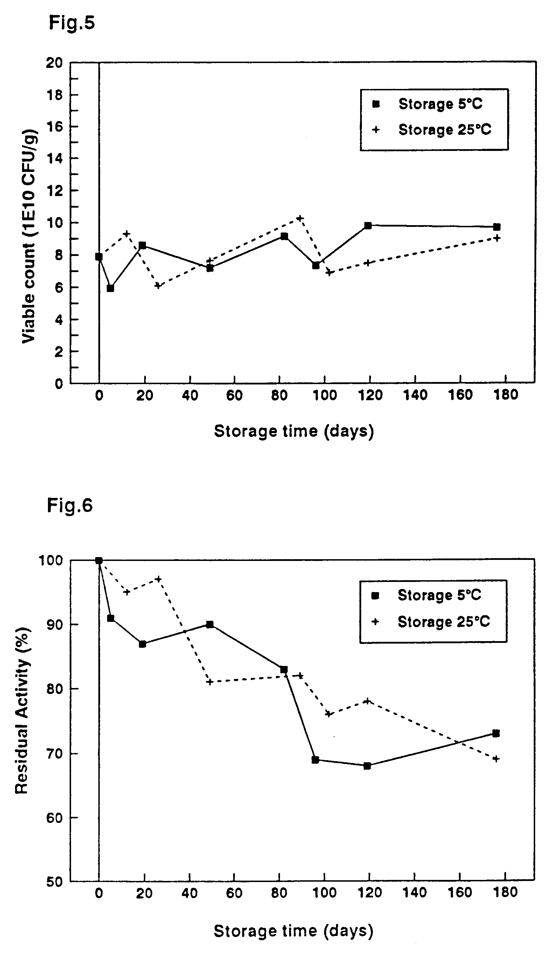

In Fig. 5 and Fig. 6, the stability of *Lb*. *plantarum* dried on Columbus flour and separated with the air-jet sieve is shown. In Fig. 5 the viable cell count in the pellets stored at 5 and 25°C for 180 days is given. The count remains stable at both temperatures. However, as can be observed in Fig. 6, the activity of *Lb*. *plantarum* decreases during storage, at both temperatures. The activity of the cells after 180 days is about 70% of the activity directly after sorption-drying and separation.

### References

Lievense L.C. et al. (1990); Measuring and modelling the glucose-fermenting activity of Lactobacillus plantarum. Appl. Microbiol. Biotechn. **32**: 669-673.
Lievense L.C. et al. (1991); Modelling the inactivation of L. plantarum during a drying process. Chem. Eng. Sci. **47**: 87-97. Lievense L.C. and Van 't Riet K. (1993); Convective drying of bacteria. I. The drying processes. In: Fiechter A. (ed) Advances in Biochemical Engineering/Biotechnology, Vol 50. Springer-Verlag, Berlin (pp. 45-63).

## Claims

1. A process for preparing a substantially dry and stable composition containing a high concentration of heat-sensitive material, comprising:
(a) contacting an aqueous concentrate of the heat-sensitive material with a food-grade water-absorbing material during sufficient time to allow transport of a large proportion of the water from the concentrate to the water-absorbing material,
(b) separating the concentrate having a decreased water content from the water-absorbing material, and
(c) collecting the separated concentrate.

2. A process according to claim 1, in which the separation of step (b) is carried out by sieving.

3. A process according to claim 2, in which a sieve is used having such sieve mesh that the particles of the water-absorbing material can pass the sieve, while the particles of the microbial cell concentrate with decreased water content are substantially retained.

4. A process according to claim 1, in which the separation of step (b) is carried out by using air classification.

5. A process according to claim 1, in which the separation of step (b) is carried out by using cyclone separation.

6. A process according to any one of claims 1-5, in which the contacting of step (a) is carried out after dropwise addition of the aqueous concentrate to the absorbent.

7. A process according to any one of claims 1-6, in which the absorbent is selected from cereal flour, milk powder and whey powder and derivatives thereof.

8. A process according to any one of claims 1-7, in which the composition contains at least 10⁷ viable microbial cells per gram.

9. A process according to claim 8, in which the microbial cells are selected from the group consisting of moulds, yeasts and bacteria.

10. A process according to claim 9, in which the bacteria are selected from the group of lactic acid bacteria and bacteria having probiotic properties.
